# EUROPÄISCHE PATENTSCHRIFT

(11) **EP 1 731 106 B1**
(45) Veröffentlichungstag und Bekanntmachung des Hinweises auf die Patenterteilung: **29.10.2008**
(21) Anmeldenummer: 06011739.7
(22) Anmeldetag: 07.06.2006
(51) Int. Cl.: A61B 17/50, A61F 11/00

(54) **Medizinisches Instrument zum Entfernen von Gegenständen aus engen Kanälen**
Medical instrument for removing objects from narrow channels
Instrument médical pour enlever des objets des canaux étroits

(30) Priorität: 09.06.2005 DE 102005026466
(43) Veröffentlichungstag der Anmeldung: 13.12.2006
(73) Patentinhaber: University of Dundee, Dundee Dundee City DD1 4HN (GB)
(72) Erfinder: Kelly, Leslie, Cupar Fife KY15 5YL (GB); Frank, Timothy G., Dr., Wormit Newport-on-Tay Fife KY16 9TY (GB); Brown, Stuart I., Dr., St. Andrews Fife KY 16 8QX (GB); Mountain, Rodney, Brunton Fife KY 15 4NB (GB); Rutherford, Ian, Dundee DD4 0RL (GB)
(74) Vertreter: Hofmeister, Frank

(56) Entgegenhaltungen:
- DE-A1- 19 514 534
- DE-U1- 29 906 201
- GB-A- 2 091 624
- US-A- 6 074 405

## Beschreibung

Die Erfindung betrifft ein medizinisches Instrument zum Entfernen von Gegenständen aus engen Kanälen, mit einem Schaft, dessen distales Ende zwischen einer im Wesentlichen gestreckten Form und einer eine hakenförmige Abwinklung aufweisenden Form verstellbar ist, wobei der Schaft mittels einer Führung in einer im Wesentlichen gestreckten Form in einen Kanal einführbar ist und der Schaft außerhalb der Führung in die die hakenförmige Abwinklung aufweisende Form überführbar ist.

Derartige hakenförmige medizinische Instrumente sind beispielsweise aus der Hals-Nasen-Ohren-Heilkunde bekannt, um mit ihnen beispielsweise Zerumen, das sogenannte Ohrenschmalz, oder andere Fremdkörper aus dem äußeren Gehörgang zu entfernen. Nachteilig bei den bekannten Instrumenten ist, dass es schwierig ist, das hakenförmige distale Ende des Schaftes hinter dem zu entfernenden Gegenstand platziert zu bekommen, um den Gegenstand anschließend mittels des Hakens aus dem Gehörgang herauszuziehen. Je nach den vorliegenden Platzverhältnissen besteht in der Praxis immer die Gefahr, dass beim Einführen des hakenförmigen Schaftes der zu entfernende Gegenstand weiter in den Gehörgang hinein gedrückt wird.

Ein gattungsgemäßes medizinisches Instrument ist beispielsweise aus der DE 195 14 534 A1 bekannt. Bei diesem bekannten medizinischen Instrument erfolgt das Verstellen der Fangschlinge über einen auf dem Schaft der Fangschlinge verschiebbar gelagerten Katheter sowie einen proximalseitig am Schaft der Fangschlinge gelagerten Torquer. Die zweihändige Bedienung von verschiebbarem Katheter einerseits und Torquer andererseits ist von der Handhabung schwierig und ermöglicht kaum ein dosiertes Einführen des Schaftes in den zu reinigenden Kanal.

Weiterhin ist aus der DE 299 06 201 U1 ein medizinisches Greif-/Schneid- und Aufnahmeinstrument bekannt, bei dem die Betätigung der Klemmbacken über ein Zylinder-Kolben-System erfolgt, wobei der Kolben lediglich dazu dient, in der Art eines Rammbocks die Klemmbacken der Zange mit starkem Druck gegen die Kraft einer die Klemmbacken zusammenpressenden Rundzugfeder auseinander zu pressen. Ein fein dosiertes Betätigen der Klemmbacken ist mit diesem Instrument kaum möglich , da das Öffnen und Schließen der Klemmbacken im wesentlichen durch die Federkraft der Rundzugfeder bestimmt wird.

Davon ausgehend liegt der Erfindung die **Aufgabe** zugrunde, ein medizinisches Instrument zum Entfernen von Gegenständen aus engen Kanälen zu schaffen, das bei einfacher Handhabung eine zuverlässige Entfernung der Gegenstände auch bei beengten Platzverhältnissen gewährleistet.

Die **Lösung** dieser Aufgabenstellung ist erfindungsgemäß dadurch gekennzeichnet, dass die Führung als Zylinder-Kolben-System ausgebildet ist, wobei der Schaft über den Kolben verschiebbar im Zylinder gelagert ist, wobei das proximale Ende des Schaftes mit dem distalen Ende des Kolbens verbunden ist.

Gemäß der Erfindung wird vorgeschlagen, dass die Führung zum Einführen des Schaftes in den Kanal als Zylinder-Kolben-System ausgebildet ist, wobei der Schaft über den Kolben verschiebbar im Zylinder gelagert ist. Diese in der Art einer Spritze ausgebildete Führung ermöglicht bei einfacher Handhabung ein zielgenaues Einführen des Schaftes. Das Verschieben des Schaftes über den Kolben kann erfindungsgemäß dadurch erleichtert werden, dass das proximale Ende des Kolbens als Daumenauflage ausgebildet ist.

Durch die erfindungsgemäße Ausgestaltung des Schaftes, wonach dessen distales Ende zwischen einer gestreckten und einer hakenförmigen Form verstellbar ist, ist es erstmalig möglich, den Schaft in einer zunächst nicht abgewinkelten Form in den zu untersuchenden Kanal einzuführen und erst innerhalb des Kanals und hinter einem zu entfernenden Gegenstand den Schaft in die abgewinkelte Hakenform zu überführen. Aufgrund dieser erfindungsgemäßen Verstellbarkeit des distalen Endes des Schaftes besteht bei einem solchermaßen ausgebildeten medizinischen Instrument nicht die Gefahr, dass der aus dem Kanal zu entfernende Gegenstand beim Einführen des Schaftes in den Kanal weiter in den Kanal hinein gestoßen wird.

Um Verletzungen des zu untersuchenden Kanals zu vermeiden, wird mit der Erfindung weiterhin vorgeschlagen, dass der Kolben über ein Federelement in eine den Schaft in den Zylinder einziehende Stellung vorgespannt ist. Sobald der Bediener des erfindungsgemäßen medizinischen Instruments keinen Druck mehr auf den Kolben ausübt, wird somit über das Federelement und den Kolben der Schaft wieder in den Zylinder hineingezogen.

Gemäß einer praktischen Ausführungsform der Erfindung wird vorgeschlagen, dass der Schaft mittels einer Führung in einer im Wesentlichen gestreckten Form in einen Kanal einführbar ist und, dass der Schaft außerhalb der Führung in die die hakenförmige Abwinklung aufweisende Form überführbar ist. Die Verwendung der Führung ermöglicht eine exakte Positionierung des Schaftes innerhalb des Kanals.

Das Überführen des distalen Endes des Schaftes in die hakenförmig abgewinkelte Form erfolgt erfindungsgemäß vorteilhafterweise selbsttätig, was durch die Verwendung eines aus einem vorgebogenen Material bestehenden Schaftes ermöglicht wird.

Als Material zur erfindungsgemäßen Ausbildung des Schaftes sind insbesondere sogenannte superelastische Werkstoffe, wie beispielsweise NiTi-Legierungen, geeignet, die die notwendige Elastizität und Steifigkeit besitzen, um einerseits in eine im Wesentlichen gestreckte Form gebogen zu werden und andererseits Dank des Memory-Effektes wieder die vorgebogene Hakenform einnehmen, wobei sie in der abgewinkelten Form ausreichend stabil sind, um den zu entfernenden Gegenstand auch entgegen einem gewissen Widerstand aus dem Kanal herausziehen zu können.

Gemäß einer alternativen Ausführungsform der Erfindung wird vorgeschlagen, dass die Führung zum Einführen des Schaftes in den Kanal als schmales Führungsrohr ausgebildet ist.

Weiterhin wird mit der Erfindung vorgeschlagen, dass das freie distale Ende des Schaftes stumpf, beispielsweise in der Form einer Schlaufe, ausgebildet ist, um auszuschließen, dass durch das zunächst in gestreckter Form in den Kanal eingeführte freie Ende des Schaftes eine Verletzung des zu untersuchenden Kanals hervorgerufen werden kann.

Schließlich wird mit der Erfindung vorgeschlagen, dass der Schaft oder die mit dem Schaft bestückte Führung über ein Spekulum in den zu untersuchenden Kanal einführbar ist. Diese trichterförmigen Instrumente sind beispielsweise aus dem HNO-Bereich bekannt, um in Körperöffnungen, wie beispielsweise den äußeren Gehörgang, eingesetzt zu werden. Das Spekulum verhindert, dass der zu untersuchende Kanal beim Einführen von Untersuchungsinstrumenten verletzt wird.

Weitere Merkmale und Vorteile der Erfindung ergeben sich anhand der zugehörigen Zeichnung, in der ein Ausführungsbeispiel eines erfindungsgemäßen medizinischen Instruments zum Entfernen von Gegenständen aus engen Kanälen nur beispielhaft dargestellt ist. In der Zeichnung zeigt:

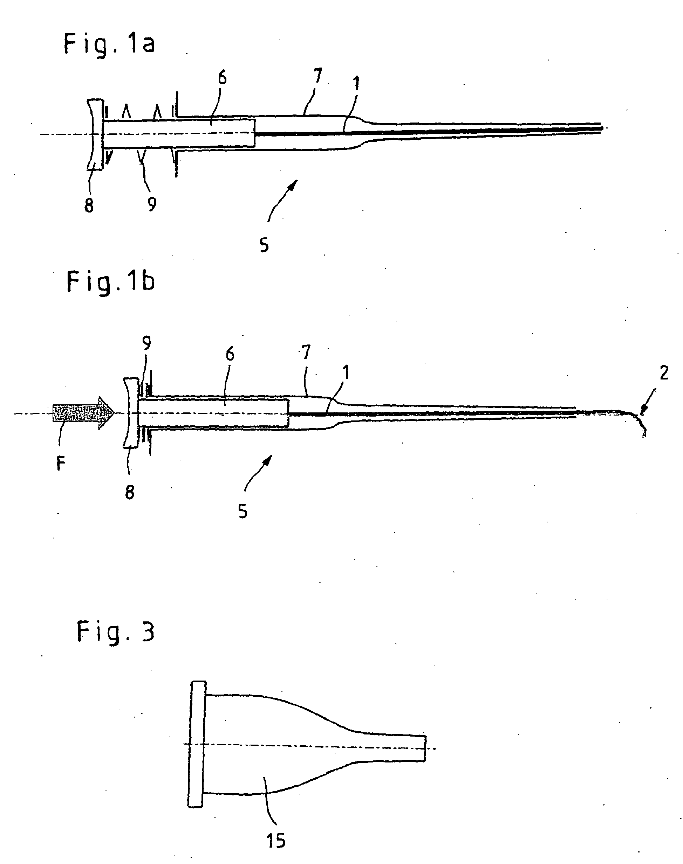

- Fig. 1a: einen Längsschnitt durch eine Ausführungsform eines erfindungsgemäßen medizinischen Instruments mit eingezogenem Schaft;
- Fig. 1 b: eine Darstellung gemäß Fig. 1a, jedoch den Schaft in der ausgefahrenen Stellung darstellend;
- Fig. 2a: eine schematische Darstellung der Verwendung des erfindungsgemäßen medizinischen Instruments gemäß Fig. 1a und 1b während des Einführens;
- Fig. 2b: eine Darstellung gemäß Fig. 2a, jedoch die Arbeitsstellung darstellend und
- Fig. 3: eine schematische Seitenansicht eines Spekulums.

Das in den Abbildungen Fig. 1a bis 2b dargestellte medizinische Instrument zum Entfernen von Gegenständen aus engen Kanälen besteht im Wesentlichen aus drahtförmigen Schaft 1, an dessen distalem Ende eine hakenförmige Abwinklung 2 zum Hintergreifen eines in einem Kanal 3 angeordneten Fremdkörpers 4 ausbildbar ist.

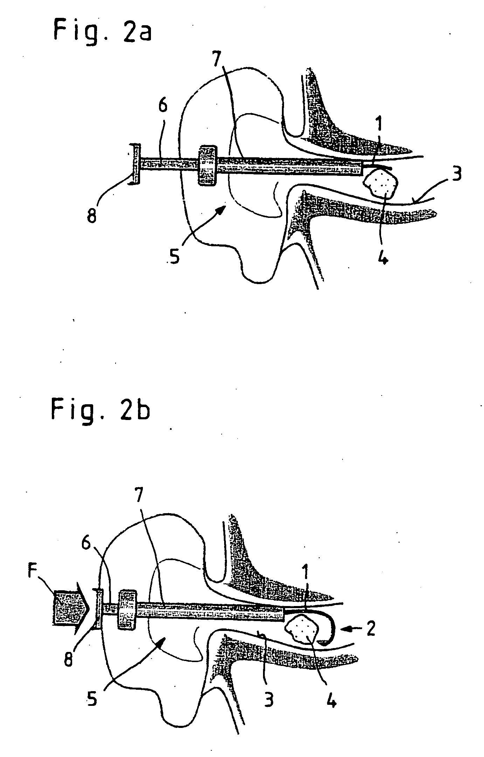

Das Einführen des Schaftes 1 in den Kanal 3, beispielsweise den äußeren Gehörgang eines Ohres, erfolgt in der Praxis mittels einer Führung 5, die bei der in den Abbildungen Fig. 1a, 1b und Fig. 2a und 2b dargestellten Ausführungsform als Zylinder-Kolben-System in der Form einer Spritze ausgebildet ist. Wie aus den Abbildungen Fig. 1 a und 1b ersichtlich, ist das proximale Ende des Schaftes 1 mit dem distalen Ende eines Kolbens 6 des Zylinder-Kolben-Systems verbunden, während der restliche Teil des Schaftes 1 in einem kanülenförmigen Zylinder 7 des Zylinder-Kolben-Systems angeordnet ist.

Zur Ausbildung der hakenförmigen Abwinklung 2 am distalen Ende des Schaftes 1 besteht der Schaft 1 aus einem elastischen Material, wie beispielsweise einer superelastischen NiTi-Legierung, das in eine hakenförmige Form vorgebogen ist. Die Elastizität des Materials erlaubt es jedoch, den Schaft in eine im Wesentlichen gestreckte Form zu überführen, um ihn mittels der Führung 5 in den zu untersuchenden Kanal 3 einführen zu können. Sobald der Schaft 1 die Führung 5 wieder verlässt, nimmt er selbsttätig wieder die vorgebogene hakenförmige Form ein.

Um ein sicheres Bedienen der als Zylinder-Kolben-System ausgebildeten Führung 5 zu gewährleisten, ist das proximale Ende des Kolbens 6 als Daumenauflage 8 ausgeformt, über die der Bediener des medizinischen Instruments mit seinem Daumen Druck in Richtung des Pfeils F auf den Kolben 6 ausüben kann, um den Schaft 1 aus dem Zylinder 7 zu drücken. Das Eindrücken des Kolbens 6 in den Zylinder 7 erfolgt gegen die Kraft eines als Druckfeder ausgebildeten Federelements 9, über das der Kolben 6 in eine den Schaft 1 in den Zylinder 7 einziehende Stellung vorgespannt ist. Dieses Federelement 9 stellt sicher, dass der Schaft 1 automatisch zurück in den Zylinder 7 der Führung 5 zurückgezogen wird, sobald der Bediener des medizinischen Instruments keine Druckkraft F mehr auf den Kolben 6 ausübt. Eine versehentliche Verletzung des Kanals 3 durch eine Nichtbedienung der Führung 5 kann so ausgeschlossen werden.

Die Handhabung des zuvor beschriebenen medizinischen Instruments zum Entfernen von Gegenständen 4 aus engen Kanälen 3 wird nachfolgend anhand des Ausführungsbeispiels der Abbildungen Fig. 1a und 1b beschrieben:

Die Abbildungen Fig. 2a und 2b zeigen schematisch die praktische Anwendung des zuvor beschriebenen medizinischen Instruments am Beispiel eines aus dem äußeren Gehörgang 3 eines Ohres zu entfernenden Fremdkörpers 4, bei dem es sich beispielsweise um verhärtetes Zerumen, das sogenannte Ohrenschmalz, handeln kann.

Um Verletzungen des Gehörgangs 3 zu vermeiden und das Einführen der Führung 5 in den Gehörgang 3 zu erleichtern, wird zunächst ein trichterförmiges Spekulum 15 in den Gehörgang 3 eingesetzt. Das in Abbildung Fig. 3 dargestellte Spekulum 15 wurde in den Abbildungen Fig. 2a und 2b aus Gründen der besseren Übersichtlichkeit weggelassen. Ebenso wurden weitere medizinische Instrumente, wie beispielsweise Otoskop zur visuellen Überwachung der Behandlung, nicht dargestellt.

Über das Spekulum 15 wird die Führung 5 in den Gehörgang 3 eingeführt, bis das distale Ende des Zylinders 7 fast den zu entfernenden Fremdkörper 4 erreicht hat. Anschließend drückt der Bediener des medizinischen Instruments über die Daumenauflage 8 den Kolben 6 in den Zylinder 7 der Führung 5, wodurch der Schaft 1 aus dem distalen Ende des Zylinders 7 herausgeschoben wird, wie dies in Fig. 2a dargestellt ist.

Der aus dem Zylinder 7 austretende Schaft 1 passiert den zu entfernenden Fremdkörper 4 und bildet bei weiterem Herausschieben des Schaftes 1 aus dem Zylinder 7 aufgrund der Vorspannung und des Memory-Effekts des Materials hinter dem Fremdkörper 4 selbsttätig die vorgeformte Abwinklung 2 aus, wie diese der Abbildung Fig. 2b zu entnehmen ist.

Zum Entfernen des Fremdkörpers 4 aus dem Gehörgang 3 muss der Bediener des medizinischen Instruments jetzt nur noch bei weiterhin eingedrücktem Kolben 6 die Führung 5 nach außen aus dem Gehörgang 3 herausziehen. Die den Fremdkörper 4 hintergreifende Abwinklung 2 nimmt diesen mit und entfernt ihn aus dem Gehörgang 3.

Neben der Auswahl eines geeignet elastischen Materials zur Ausbildung des Schaftes 1, um ein wiederholtes Strecken und Zurückbiegen des distalen Endes des Schaftes 1 zu ermöglichen, ist das Material hinsichtlich der Materialsteifigkeit und/oder Materialstärke so auszuwählen, dass die den zu entfernenden Fremdkörper 4 hintergreifende Abwinklung 2 sich nicht sofort zurück biegt, wenn durch den Fremdkörper 4 eine Widerstandskraft auf die hakenförmige Abwinklung 2 ausgeübt wird.

Ein solchermaßen ausgebildetes medizinisches Instrument zum Entfernen von Gegenständen 4 aus engen Kanälen 3 zeichnet sich dadurch aus, dass es bei einfachem Aufbau und einfacher Handhabung ein zuverlässige Entfernung der Fremdkörper 4 aus dem zu untersuchenden Kanal 3 gewährleistet. Da der Schaft 1 über die Führung 5 in gestreckter Form, das heißt ohne Abwinklung 2, in den Kanal 3 eingeführt werden kann und sich die zum Ergreifen des Fremdkörpers 4 dienende Abwinklung 2 erst hinter dem Fremdkörper 4 ausbildet, besteht nicht die Gefahr, dass der Fremdkörper 4 beim Einführen des Schaftes 1 in den Kanal 3 weiter in den Kanal 3 hineindrückt wird.

### Bezugszeichenliste

- 1: Schaft
- 2: Abwinklung
- 3: Kanal/Gehörgang
- 4: Fremdkörper
- 5: Führung
- 6: Kolben
- 7: Zylinder
- 8: Daumenauflage
- 9: Federelement
- 15: Spekulum

- F: Druckkraft

## Patentansprüche

1. Medizinisches Instrument zum Entfernen von Gegenständen aus engen Kanälen, mit einem Schaft (1), dessen distales Ende zwischen einer im Wesentlichen gestreckten Form und einer eine hakenförmige Abwinklung (2) aufweisenden Form verstellbar ist, wobei der Schaft (1) mittels einer Führung (5) in einer im Wesentlichen gestreckten Form in einen Kanal (3) einführbar ist und der Schaft (1) außerhalb der Führung (5) in die die hakenförmige Abwinklung (2) aufweisende Form überführbar ist,
**dadurch gekennzeichnet,**
**dass** die Führung (5) als Zylinder-Kolben-System ausgebildet ist, wobei der Schaft (1) über den Kolben (6) verschiebbar im Zylinder (7) gelagert ist, wobei das proximale Ende des Schaftes (1) mit dem distalen Ende des Kolbens (6) verbunden ist.

2. Medizinisches Instrument nach Anspruch 1, **dadurch gekennzeichnet, dass** der Kolben (6) über ein Federelement (9) in eine den Schaft (1) in den Zylinder (7) einziehende Stellung vorgespannt ist.

3. Medizinisches Instrument nach Anspruch 1 oder 2, **dadurch gekennzeichnet, dass** das proximale Ende des Kolbens (6) als Daumenauflage (8) ausgebildet ist.

4. Medizinisches Instrument nach einem der Ansprüche 1 bis 3 **dadurch gekennzeichnet, dass** das distale Ende des Schaftes (1) selbsttätig die die hakenförmige Abwinklung (2) aufweisende Form einnimmt.

5. Medizinisches Instrument nach einem der Ansprüche 1 bis 4, **dadurch gekennzeichnet, dass** der Schaft (1) aus einem vorgebogenen elastischen Material besteht.

6. Medizinisches Instrument nach Anspruch 5, **dadurch gekennzeichnet, dass** der Schaft (1) aus einem elastischen Material, insbesondere einer NiTi-Legierung, besteht.

7. Medizinisches Instrument nach einem der Ansprüche 1 bis 6, **dadurch gekennzeichnet, dass** die Führung (5) als schmales Führungsrohr ausgebildet ist.

8. Medizinisches Instrument nach einem der Ansprüche 1 bis 7, **dadurch gekennzeichnet, dass** das freie distale Ende des Schaftes (1) stumpf ausgebildet ist.

9. Medizinisches Instrument nach einem der Ansprüche 1 bis 8, **dadurch gekennzeichnet, dass** der Schaft (1) über ein Spekulum (15) in den Kanal (3) einführbar ist.

## Claims

1. Medical instrument for removing objects from narrow channels, with a shaft (1) whose distal end is adjustable between a substantially linear form and a form having a hook-shaped curvature (2), the shaft (1) being able to be inserted in a substantially linear form into a channel (3) by means of a guide (5), and the shaft (1) being able to be converted outside the guide (5) into the form having the hook-shaped curvature (2), **characterized in that** the guide (5) is designed as a cylinder/piston system, the shaft (1) being mounted in the cylinder (7) so as to be displaceable via the piston (6), and the proximal end of the shaft (1) being connected to the distal end of the piston (6).

2. Medical instrument according to Claim 1, **characterized in that** the piston (6) is pretensioned, via a spring element (9), into a position drawing the shaft (1) into the cylinder (7).

3. Medical instrument according to Claim 1 or 2, **characterized in that** the proximal end of the piston (6) is designed as a thumb support (8).

4. Medical instrument according to one of Claims 1 to 3, **characterized in that** the distal end of the shaft (1) automatically adopts the form having the hook-shaped curvature (2).

5. Medical instrument according to one of Claims 1 to 4, **characterized in that** the shaft (1) is made of a pre-bent elastic material.

6. Medical instrument according to Claim 5, **characterized in that** the shaft (1) is made of an elastic material, in particular a NiTi alloy.

7. Medical instrument according to one of Claims 1 to 6, **characterized in that** the guide (5) is designed as a narrow guide tube.

8. Medical instrument according to one of Claims 1 to 7, **characterized in that** the free distal end of the shaft (1) is blunt.

9. Medical instrument according to one of Claims 1 to 8, **characterized in that** the shaft (1) can be inserted into the channel (3) via a speculum (15).

## Revendications

1. Instrument médical pour retirer des objets de canaux étroits, comprenant un corps allongé (1) dont l'extrémité distale peut être déplacée entre une forme sensiblement étendue et une forme présentant un coude (2) en forme de crochet, le corps allongé (1) pouvant, au moyen d'un guidage (5), être introduit dans un canal (3) en étant dans une forme sensiblement étendue, et le corps allongé (1) pouvant être transféré, à l'extérieur du guidage (5), à la forme présentant le coude (2) en forme de crochet,
**caractérisé**
**en ce que** le guidage (5) est réalisé sous la forme d'un système à cylindre et piston, le corps allongé (1) étant monté dans le cylindre (7) de manière à pouvoir y coulisser par l'intermédiaire du piston (6), et l'extrémité proximale du corps allongé (1) étant reliée à l'extrémité distale du piston (6).

2. Instrument médical selon la revendication 1, **caractérisé en ce que** le piston (6) est précontraint, par l'intermédiaire d'un élément de ressort (9), dans une position pour laquelle le corps allongé (1) est tiré à l'intérieur du cylindre (7).

3. Instrument médical selon la revendication 1 ou 2, **caractérisé en ce que** l'extrémité proximale du piston (6) est réalisée sous la forme d'un appui de pouce (8).

4. Instrument médical selon l'une des revendications 1 à 3, **caractérisé en ce que** l'extrémité distale du corps allongé (1) prend automatiquement ou d'elle-même la forme présentant le coude (2) en forme de crochet.

5. Instrument médical selon l'une des revendications 1 à 4, **caractérisé en ce que** le corps allongé (1) est réalisé en un matériau élastique pré-coudé ou précintré.

6. Instrument médical selon la revendication 5, **caractérisé en ce que** le corps allongé (1) est réalisé en un matériau élastique, notamment en un alliage NiTi.

7. Instrument médical selon l'une des revendications 1 à 6, **caractérisé en ce que** le guidage (5) est réalisé sous la forme d'un tube de guidage étroit.

8. Instrument médical selon l'une des revendications 1 à 7, **caractérisé en ce que** l'extrémité distale libre du corps allongé (1) est de configuration émoussée.

9. Instrument médical selon l'une des revendications 1 à 8, **caractérisé en ce que** le corps allongé (1) peut être introduit dans le canal (3) par l'intermédiaire d'un speculum (15).
